# EUROPEAN PATENT SPECIFICATION

(11) **EP 0 716 688 B1**
(45) Date of publication and mention of the grant of the patent: **24.09.2003**
(21) Application number: 94925419.7
(22) Date of filing: 29.07.1994
(51) Int. Cl.: C12N 1/20, C12P 19/26, C08B 37/08, A61K 31/36

(54) **CULTURE MEDIUM AND PROCESS FOR THE PREPARATION OF HIGH MOLECULAR WEIGHT HYALURONIC ACID**
KULTURMEDIUM UND VERFAHREN ZUR HERSTELLUNG VON HYALURONSÄURE MIT HOHEM MOLEKULARGEWICHT
MILIEU DE CULTURE ET PROCEDE DE PREPARATION D'ACIDE HYALURONIQUE A HAUT POIDS MOLECULAIRE

(30) Priority: 30.07.1993 IT PD930164
(43) Date of publication of application: 19.06.1996
(73) Proprietor: FIDIA ADVANCED BIOPOLYMERS S.R.L., 72100 Brindisi (IT)
(72) Inventor: CAZZOLA, Flavia, I-34170 Gorizia (IT); O'REGAN, Michael, I-35100 Padova (IT); CORSA, Vincenza, I-72100 Brindisi (IT)
(74) Representative: Rostovanyi, Peter
(86) International application number: EP9402537
(87) International publication number: WO95004132

(56) References cited:
- EP-A- 0 244 757
- EP-A- 0 266 578
- WO-A-84/03302
- WO-A-86/04355
- WO-A-92/08799
- US-A- 2 975 104
- US-A- 4 897 349
- Week 7703, Derwent Publications Ltd., London, GB; AN 77-04750Y & JP,A,51 139 692 (CHUGAI PHARMACEUTICAL KK) 3 February 1976
- DATABASE WPI Week 7703, Derwent Publications Ltd., London, GB; AN 77-04745Y & JP,A,51 139 685 (HITACHI CHEMICAL KK) 3 December 1976
- CHEMICAL ABSTRACTS, vol. 107, no. 11, 14 September 1987, Columbus, Ohio, US; abstract no. 95297s, page 573 ;column R ; & JP,A,6 251 999 (KYOWA HAKKO KOGYO) 6 March 1987
- CHEMICAL ABSTRACTS, vol. 107, no. 3, 20 July 1987, Columbus, Ohio, US; abstract no. 21971k, page 491 ;column R ; & JP,A,6 232 893 (KYOWA HAKKO KOGYO CO. LTD) 12 February 1987

## Description

### Background of the Invention

### Field of the Invention

The present invention relates to a method for the production of high molecular weight hyaluronic acid by fermentation techniques, employing streptococci and other bacteria capable of producing hyaluronic acid, and a semidefined, cost-effective culture medium.

### Description of Related Art

Hyaluronic acid (HA) is a highly viscous glycosaminoglycan comprising alternating, repetitive units of glucuronic acid and N-acetyl-glucosamine joined by β1-3 and β1-4 glycoside bonds. Hyaluronic acid is present in various animal connective tissues, such as the skin and cartilage, in the umbilical cord, the synovial fluid, aqueous humor, and in cockscombs.

The role of hyaluronic acid in the skin and in the cartilage is to retain water and lend elasticity and tone to the tissues, while in the joint fluids, it acts as a lubricant and protects the cells from physical shocks from the outside, and from bacterial infections. Due to its highly hydrophilic nature, hyaluronic acid is an ideal constituent in the preparation of cosmetic lotions. Highly purified solutions of HA are also used as support media in eye surgery (U.S. Patent No. 4,141,973) or as a substitute fluid in the joints in the case of particular pathological conditions.

The production of high molecular weight hyaluronic acid, which has greater viscoelastic properties than that of low molecular weight fractions, is therefore of considerable interest, especially in view of the necessity to absorb major variations in pressure such as those which occur *in vivo* in the joints.

The extraction of hyaluronic acid from animal sources presents various disadvantages due to the limited availability of raw material, the difficulty of extracting large quantities of high molecular weight hyaluronic acid from animal tissues, where only traces of it are present, often in the form of complexes with proteins or other mucopolysaccharides, and the danger of viral contamination. For these reasons, many researchers have turned to the production of HA from microbiological sources.

The production of HA by streptococci was first demonstrated by Forrest et al., J. Biol. Chem. 118:61 (1937), and subsequently by other researchers, e.g., Roseman et al., J. Biol. Chem. 203:213 (1953); Pierce et al., Proc. Soc. EXP. Biol. Med. 87:50 (1954); U.S. Patent No. 2,975,104; and Sunghara et al., J. Biol.Chem. 254:6252 (1979). These workers demonstrated that the hyaluronic acid present in the capsules of many strains of group A and group C streptococci is the same as that from animal sources. These microorganisms can assimilate glucose or other sources of carbon and, under particular environmental conditions, produce hyaluronic acid as a secondary metabolite. The synthesis of hyaluronic acid by streptococci is, however, influenced by many genetic and nutritional factors.

Streptococci are among the most demanding bacteria from a nutritional point of view. The literature describes many types of culture media that favor the production of hyaluronic acid by these microorganisms, which normally include carbohydrates, amino acids, vitamins, inorganic salts, and some growth factors. Generally, glucose is used as a carbon source, while the nitrogen source can be organic or inorganic, including peptone, yeast extract, hydrolyzed casein products, hydrolyzed soybean products, amino acids, ammonium sulfate, ammonium chloride, ammonium phosphate, urea, glutamine, etc. Certain inorganic salts may also be added to the medium, such as sulfates, hydrochlorides, carbonates, nitrates, phosphates, and acetates of calcium, potassium, sodium, magnesium, manganese, iron, copper, and zinc. Lastly, the addition of vitamins and amino acids can also prove advantageous to bacterial growth.

Some nutritive media specific for streptococci are already sold commercially, such as Brain Heart Infusion and Todd-Hewitt Broth. Others have been described in patents relating to hyaluronic acid production by fermentation, such as U.S. Patent No. 4,517,295, U.S. Patent No. 2,975,104, U.S. Patent No. 4,784,990, U.S. Patent No. 4,897,349, and European Patent No. 0 244 757. Many of these media, however, are disadvantageous in that they require expensive components, and are therefore unsuitable for industrial purposes. Hence, the HA product obtained via fermentation using these complex, expensive media is not economically advantageous compared to that obtained from animal sources.

Another problem associated with the production of hyaluronic acid from bacterial sources is the use, in some cases, of strains of streptococci which are pathogenic to humans, such as *S. pyogenes* (U.S. Patent No. 4,517,295).

However, the production of hyaluronic acid by fermentation, apart from facilitating the obtainment of large quantities of polymer with a higher molecular weight than that obtained from animal tissues, also enables purification of HA by less complicated methods than those required for other starting materials. The known purification procedures, which have been described in numerous patents, allow the isolation of high molecular weight hyaluronic acid, often with a high level of purity. These methods usually require large quantities of solvents or other toxic products, such as cetylpyridine chloride, therefore also requiring numerous passages before producing highly purified hyaluronic acid for use in the pharmaceutical field. All these factors creates cost- and environment-related problems.

### Therapeutic Uses of Hyaluronic Acid

The notable biocompatible and biodegradable characteristics of HA have been determining factors in its use in the field of viscosurgery. Indeed, hyaluronic acid, in its highly polymeric form, which reaches a molecular weight of 4-6 million, is present at various concentrations in the connective tissue matrix of all primates, and its use in viscosurgery therefore causes only a temporary increase in concentration compared to that of the native hyaluronic acid.

In certain surgical operations, HA can protect tissues by absorbing shocks and lubricating cellular layers open to mechanical damage by surgical instruments or implants. It represents the medium in which surgery is performed, avoids the formation of tissue adherence, and allows fragments of tissue and blood clots to be removed. Besides this protective effect during surgery, hyaluronic acid, in its role as a molecular barrier, can be used as an implant between two tissue surfaces that must be kept apart in order to function properly.

Another application of hyaluronic acid is linked with its viscoelastic properties. In some pathological conditions, e.g., arthritis and chronic or acute arthrosis, some tissues lose viscoelasticity, and this loss is characterized by both a decrease in molecular weight and in the concentration of endogenous hyaluronic acid. "Viscosupplementation" is a therapeutic process to restore or increase normal rheological properties by introducing a viscous medium such as hyaluronic acid into the tissue compartment.

The biological role of hyaluronic acid in tissue repair has also been amply described in the literature. The addition of exogenous HA can play an important role in accelerating or modifying tissue repair, facilitating the movement of inflammatory, mesenchymal, and epithelial cells at the tissue repair site. Tissue repair consists of a complex series of events involving various types of cells, with the ultimate aim of forming a scar. This last stage of the repair process, albeit necessary, often represents an esthetic problem. It is very important, therefore, to be able to reduce scar formation.

### Summary of The Invention

It is therefore an object of the present invention to provide a culture medium, comprising a mixture of two different nitrogen sources, i.e., a soybean meal digest as well as a peptone mixture, and a carbon source. Said soybean meal digest is HY-SOY, said peptone mixture is SPECIAL PEPTONE, and said carbon source is glucose. Said soybean meal digest or HY-SOY is present at a concentration of between about 10 g/l and about 30 g/l, preferably about 20 g/l, said. peptone mixture or SPECIAL PEPTONE is present at a concentration of between about 5 g/l and about 15 g/l, preferably about 10 g/1, and the carbon source or glucose is present at a concentration of between about 10 g/l and about 60 g/l, preferably about 30g/l. The pH of the medium can be in the range of from about 6.5 to about 7.5. The culture medium can further comprise a free radical scavenger, at a concentration of from about 0.01 g/l to about 1 g/l, preferably about 0.1 g/l.

It is another object of the present invention to provide a process for producing hyaluronic acid, comprising cultivating a bacterial strain capable of producing hyaluronic acid in a culture medium comprising a soybean meal digest, a peptone mixture, and a carbon source, as described above, under conditions which promote the formation of hyaluronic acid by said bacterial strain, and recovering said hyaluronic acid. The bacterial strain can be a group A or group C strain of *Streptococcus.* More particularly, the streptococcal strain can be a strain of S. *equi, S. equisimilis, S. dysgalactiae, S. pyogenes,* or S. zooepidemicus. S. equi 68222 has been employed in the Examples *infra.* Most preferably, glucose is present at a concentration of 30 g/l, and the free radical scavenger is present at a concentration of from about 0.01 g/l to about 1 g/l, preferably about 0.1 g/l. Bacterial growth can be carried out by cultivation under aerobic conditions by introducing sterile air at a rate of between about 0.1 to about 4 vvm, preferably 2 vvm, and shaking vigorously at a rate of between about 200 rpm and about 900 rpm, preferably about 600 rpm.

### < Further aspects >

Further, a process for producing hyaluronic acid is discosed herein comprising cultivating a streptococcal strain capable of producing hyaluronic acid in a culture medium comprising a soybean meal digest or HY-SOY at a concentration of between about 10g/l and about 30 g/l, preferably about 20 g/l, a peptone mixture or SPECIAL PEPTONE at a concentration of between about 5 g/l and about 15 g/l, preferably about 10 g/l, or yeast extract at a concentration of between about 5 g/l and about 15 g/l, preferably about 10 g/l, glucose at a concentration of between about 10 g/l and about 60 g/l, preferably about 30 g/l, and a free radical scavenger at a concentration of from about 0.01 g/l to about 1 g/l, preferably 0.1 g/l, under conditions which promote the formation of hyaluronic acid by said streptococcal strain, and recovering said hyaluronic acid.

Still further, a process for purifying hyaluronic acid produced by bacterial cells grown in a culture medium is disclosed herein, comprising killing bacterial cells present in said culture medium; releasing any hyaluronic acid adhering to said bacterial cells by treatment thereof with a surfactant; removing bacterial cells by prefiltering said culture medium through a filter having a porosity of from about 0.1 µm to about 10 µm; removing any remaining bacterial cells by microfiltration of said culture medium through a filter having a porosity of about 0.45 µm; dialyzing said culture medium against distilled water; and concentrating the dialyzed culture medium.

Still further, a process for producing hyaluronic acid, comprising cultivating a streptococcal strain capable of producing hyaluronic acid in a culture medium is disclosed herein comprising. a soybean meal digest or HY-SOY at a concentration of between about 10g/l and about 30 g/l, preferably about 20 g/l, a peptone mixture or SPECIAL PEPTONE at a concentration of between about 5 g/l and about 15 g/l, preferably about 10 g/l, or yeast, extract at a concentration of between about 5 g/l and about 15 g/l, preferably about 10 g/l, glucose at a concentration of between about 10 g/l and about 60 g/l, preferably about 30 g/l, and a free radical scavenger at a concentration of from about 0.01 g/l to about 1 g/l, preferably about 0.1 g/l, under conditions which promote the formation of hyaluronic acid by said streptococcal strain; killing bacterial cells present in said culture medium by treating said cells with a bactericide selected from formaldehye, an alcohol, chloroform, and acetone; releasing any hyaluronic acid adhering to said bacterial cells by treatment thereof with a surfactant; removing bacterial cells by prefiltering said culture medium through a filter having a porosity of from about 0.1 µm to about 10 µm; removing any remaining bacterial cells by microfiltration of said culture medium through a filter having a porosity of about 0.45 µm; dialyzing said culture medium against distilled water; and concentrating the dialyzed medium.

Still further, a hyaluronic acid fraction having a molecular weight in the range of from about 2X10⁶ Daltons to about 3X10⁶ Daltons, preferably about 2X10⁶ Daltons, is disclosed herein Hyaluronic acid having a molecular weight in the range of from about 2X10⁶ Daltons to about 3X10⁶ Daltons, preferably about 2X10⁶ Daltons, may be used in the manufacture of a medicament for treating wounds or surgical incisions to avoid the formation of hypertrophic or keloid scars.

Further, a pharmaceutical composition is disclosed herein, comprising hyaluronic acid having a molecular weight in the range of from about 2X10⁶ Daltons to about 3X10⁶ Daltons, preferably about 2X1⁰⁶ Daltons, and a pharmaceutically acceptable carrier, diluent, or excipient.

Still further, a method for treating a wound or surgical incision to avoid hypertrophic scar.or keloid formation is disclosed herein, comprising administering to said wound or surgical incision an effective amount of hyaluronic acid having a molecular weight in the range of from about 2X10⁶ Daltons to about 3X10⁶ Daltons, preferably about 2X10⁶ Daltons.

Via the use of the medium and method of the present invention, hyaluronic acid having a molecular weight in the range of from about 2 to about 3.1 X 10⁶ Daltons can be obtained in a yield of about 2 g/l.

The present inventors have therefore provided a method of producing high molecular weight hyaluronic acid from bacterial sources using a new and cost-efficient culture medium which improves the HA/cell ratio, thus facilitating the separation of the bacteria from the culture broth, and thereby, the whole purification process.

The method for the isolation and purification of hyaluronic acid released into the culture medium provided herein results in a good yield, is easily reproducible, produces a therapeutically acceptable product, is economical, and reduces environmental problems.

The high molecular weight hyaluronic acid produced via the present process can be used for the modulation of wound healing.

Further scope of the applicability of the present invention will become apparent from the detailed description provided below. However, it should be understood that the detailed description and specific examples, while indicating preferred embodiments of the invention, are given by way of illustration only since various changes and modifications within the spirit and scope of the invention will become apparent to those skilled in the art from this detailed description.

### Detailed Description of the Invention

The following detailed description of the invention is provided to aid those skilled in the art in practicing the present invention. Even so, the following detailed description should not be construed to unduly limit the present invention, as modifications and variations in the embodiments herein discussed may be made by those of ordinary skill in the art without departing from the spirit or scope of the present inventive discovery.

The contents of each of the references cited herein are herein incorporated by reference in their entirety.

Streptococci of the A and C groups, according to Lancefield's classification, produce a hyaluronic acid capsule, the composition of which is identical to that of the polymer present in mammalian connective tissues (R. M. Krause (1972) "The Streptococcal Cell: Relationship of Structure to Function and Pathogenesis," in L.W. Wannamaker and J. M. Matsen, eds., Streptococci and Streptococcal Diseases, Academic Press Inc., New York, pp. 3-18) . A large variety of streptococci in the A and C groups are capable of producing high concentrations of HA. The microorganisms that can be used in the process of the present invention include S. dysgalactiae, S. equi, S. equisimilis, S. pyogenes, and S. zooepidenacus, which belong to the C group, and which are therefore not pathogenic to man. The prefered species is S. equi, and in particular the *S. equi* 68222 strain from the Belfanti Institute in Milan. This strain has not been genetically engineered, and is not, therefore, subject to the disadvantage of instability, which is characteristic of genetically manipulated strains. *S.* equi 68222 produces hyaluronic acid under conditions of aerobiosis; the carbon source can be glucose.

It is well known that streptococci are, from a nutritional point of view, some of the most demanding bacteria (Terleckyl et al., Infection and Immunity 649-655, 1975; Milligan et al. J. of Clinical Microbiol. 28-33, 1978). Various culture media have been described in the literature that promote the growth of these microorganisms and their production of hyaluronic acid. Such media normally include carbohydrates, amino acids, vitamins, and inorganic salts, as well as various growth factors. The influence of the carbon source, nitrogen, and inorganic compounds on bacterial growth and metabolism is very important for these exopolysaccharide-producing microorganisms (Robinson et al. Biotech. and Bioeng. XXVI:1409-1417, 1984; MacFarlane et al. J. of Appl . Bacterial. 68:179-187, 1990).

With respect to the most appropriate source of carbon for the production of HA, the most widely used is glucose, which is also used according to the invention, although other sugars can be successfully employed. These include, for example, sucrose, lactose, blackstrap molasses, and starch hydrolysates. Indeed, apart from being a source of energy for cell growth, these can also be used for the synthesis of the single monomers that comprise the HA polymer.

Various organic and inorganic nitrogen sources can be used, including yeast extract, peptones, casein hydrolysates, soybean protein, ammonium sulfate, ammonium chloride, ammonium phosphate, etc. According to the invention a peptone mixture is used.

Apart from these two main components, inorganic salts can be added to the culture medium, such as various sulfates, carbonates, phosphates, calcium chloride, potassium, magnesium, sodium, manganese, zinc, iron, etc., which enhance growth and which can sometimes intervene as enzyme cofactors involved in hyaluronic acid synthesis.

The present inventors have therefore devised a culture medium which facilitates the production of high molecular weight hyaluronic acid in good yield, and which is economical to use, with a view toward production on an industrial scale. This medium results in a better hyaluronic acid/cell ratio as compared to that obtained via bacterial culture on commercial media such as Brain Heart Infusion or on various types of media described in the patent literature. A high HA/cell ratio facilitates the initial stage of purification, which consists of the separation of the bacterial cells from the culture broth containing the biopolymer.

### EXAMPLE 1

### Comparison of Bacterial Growth and Hyaluronic Acid Production on Various Media

The following semidefined medium, designated HYP, was prepared in distilled water:

| **HYP Medium** | |
|---|---|
| HY-SOY | 10 g/l |
| SPECIAL PEPTONE | 10 g/l |
| Glucose | 30 g/l |

HY-SOY is a commercial product of Sheffield Products-QUEST International. It is a water-soluble plant nitrogen source obtained from a papaic digest of soybean meal. Owing to its high carbohydrate level, this nitrogen source stimulates rapid and heavy growth of microorganisms.

SPECIAL PEPTONE (POLYPEPTONE) is a commercial product of UNIPATH. It is a peptone mixture composed of meat, plant, and yeast extract, and contains a large variety of available peptides, minerals, vitamins, nucleotides, and other organic compounds.

Equivalents of SPECIAL PEPTONE which can be employed in the present invention include PEPTONE BACTERIOLOGICAL TECHNICAL from Difco, PEPTONE FROM MEAT from Serva, and PRIMATONE from Sheffield Products.

YEAST EXTRACT from Difco and other commercially available equivalents thereof, can be employed in HYP medium in place of SPECIAL PEPTONE. YEAST EXTRACT is the water-soluble portion of autolyzed yeast, standardized for microbiological use.

A glucose stock solution was prepared by filtration through a 0.22 micron filter. The HY-SOY and SPECIAL PEPTONE were autoclaved at 121°C for 20 minutes. Appropriate amounts of both sterilized solutions were mixed, and the pH of the medium was adjusted to a value of about 7.

The growth and production of HA by *S. equi* 68222 in this culture medium was compared to that using the same microorganism cultivated in a commercially available medium (Brain Heart Infusion, BHI) or media described in various patents (European Patent Application 0 244 757; U.S. Patent No. 4,784,990; and European Patent Application 0 266 578) as follows:

| **Brain Heart Infusion (BHI)** | |
|---|---|
| Calf Brains, Infusion from | 200 g/l |
| Beef heart, Infusion from | 250 g/l |
| Proteose peptone | 10 g/l |
| Bacto dextrose | 2 g/l |
| NaCl | 5 g/l |
| Disodium phosphate | 2.5 g/l |

| **Medium in U.S. Patent 4,784,990** | |
|---|---|
| Casein hydrolysate | 20 g/l |
| Yeast extract | 10 g/l |
| NaCl | 2 g/l |
| MgSO₄.7H₂O | 1.5 g/l |
| K₂HPO₄ | 2.5 g/l |
| Glucose | 5 g/l |

| **Medium in EP 0 266 578** | |
|---|---|
| Peptone | 15 g/l |
| Yeast extract | 5 g/l |
| KH₂PO₄ | 10 g/l |
| CH₃COONa . 3H₂O | 5 g/l |
| NaHCO₃ | 3 g/l |
| MgSO₄. 7H₂O | 0.2 g/l |
| FeSO₄. 7H₂O | 0.01 g/l |
| MnCl₂.4H₂O | 0.01 g/l |
| CaCl₂ | 0.05 g/l |
| Glucose | 30 g/l |

| **Medium in EP 0 244 757 (KBS Medium)** | |
|---|---|
| Polypeptone | 15 g/l |
| Yeast extract | 5 g/l |
| KH₂PO₄ | 2 g/l |
| MgSO₄.7H₂O | 1 g/l |
| CaCl₂ | 0.1 g/l |
| Glucose | 60 g/l |

A loopful of *S. equi* 68222 was inoculated into 100 ml of each of these different media, previously sterilized, in 250 ml Erlenmeyer flasks. The cultures were incubated in duplicate overnight at 37°C while being shaken at 200 rpm. After measuring the optical density of each culture at 600 nm, the contents of each flask were diluted with fresh medium until an optical density of about 0.1 was obtained, so as to obtain a final volume of 200 ml in a 1 liter flask. The growth temperature was maintained at 37°C during vigorous shaking (200 rpm) at a pH of 7. When the pH of the medium moved outside the appropriate range, it was brought back to the appropriate value by adding a suitable quantity of acid or base, e.g., aqueous lN HC1 or aqueous IN NaOH.

During cell culture, growth was monitored by taking optical density readings at 600 nm, while the pH was monitored using phenol red, and was maintained at a value of about 7. At the end of fermentation, i.e., when the bacteria had stopped growing, cell growth and hyaluronic acid production were measured. The results are shown in Table 1.

**TABLE 1**

| **CULTURE MEDIUM** | **O.D. (600 mm)** | **HA (g/L)** | **HA/cell** |
|---|---|---|---|
| HYP | 3,5 | 136 | 0,38 |
| BHI | 5,4 | 1 | 0,18 |
| Patent N° 0,244,757 | 5,3 | 0,8 | 0,15 |
| Patent N° 4,784,990 | 2,60 | 0,38 | 0,14 |
| Patent N° 0,266,578 | 6 | 0,76 | 0,12 |

The quantity of hyaluronic acid produced by fermenting *S. equi* 68222 in HYP medium is greater than that obtained in Brain Heart Infusion and the other tested media. Furthermore, the cell concentration is considerably lower, accounting for the better HA/cell ratio and the easier separation of the bacteria from the culture broth.

The extreme simplicity of the present medium and lower cost of the nitrogen source employed therein compared to that of the nitrogen sources used in the comparative media make HYP more cost-efficient and competitive, especially for industrial applications.

### EXAMPLE 2

### Effect of Glucose Concentration, Aeration, and Free Radical Scavengers on Bacterial Growth and Hyaluronic Acid Production

By modifying the culture parameters, it is possible to increase the production of microbial polysaccharides. The use of batch or continuous culture makes it possible to apply different physiological pressures on the growth of microorganisms and on polysaccharide synthesis.

The effect of different concentrations of glucose on cell growth and hyaluronic acid production was determined. Cell growth, glucose consumption, HA production, efficiency in converting glucose into HA, and HA/cell ratio were analyzed employing glucose concentrations of 0, 5, 15, 30, and 60 g/l in the culture medium.

A loopful of *S.equi* 68222 was resuspended in 200 ml of HYP culture medium in the absence of glucose in a 1 liter Erlenmeyer flask. The culture was shaken at 200 rpm at 37°C overnight. The next day, the culture was used to inoculate ten 500 ml flasks. A quantity of the inoculum was diluted with fresh medium to obtain an optical density 0.1 in a total volume of 100 ml. Glucose was added to the flasks in final amounts of 0 g/l, 5g/l, 15 g/l, 30 g/l, and 60 g/l. Each experiment was performed in duplicate. The flasks were incubated at 37°C with shaking at 200 rpm. Optical density, glucose consumption, and HA production were monitored during the growth stage; at the end of fermentation, the cell concentration was measured in terms of grams of dry weight.

As can be seen from the results reported in Table 2, the highest values for HA production are obtained using 30 g/l and 60 g/l of glucose, even though at 60 g/l growth seems slightly slower; while the cell concentration at steady state is similar in the two cases. On the other hand, by decreasing the glucose concentration in the medium, there is a simultaneous decrease in the production of HA. A glucose concentration of about 30 g/1 therefore seems to be optimal for HA production by *S. equi* 68222.

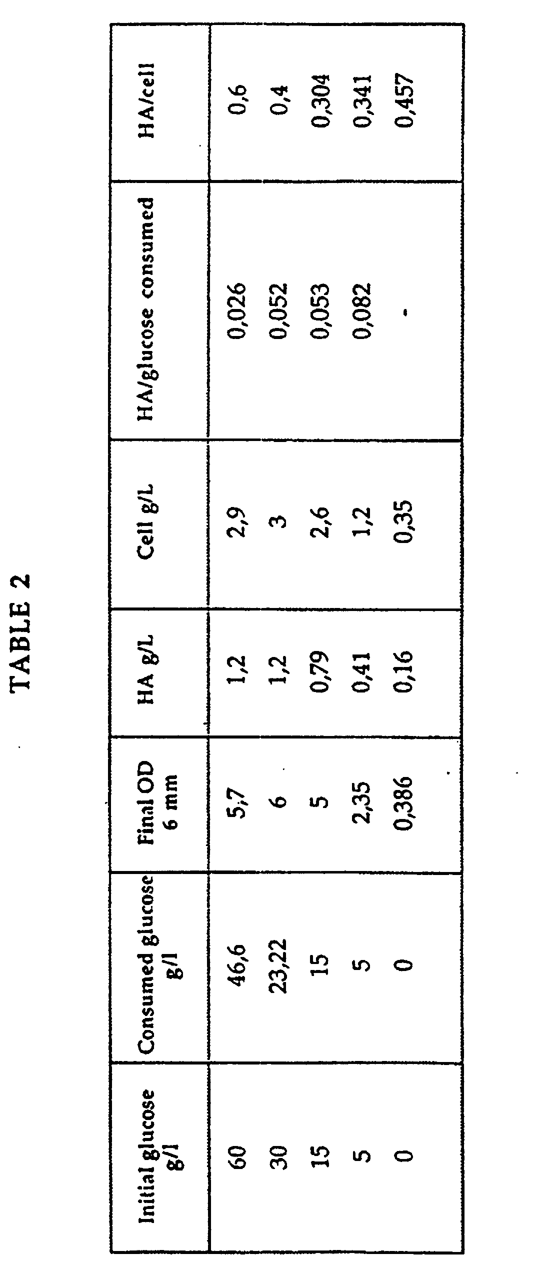

The effect of oxygenation on bacterial growth and HA production was also assessed. Eight 1 liter Erlenmeyer flasks containing, in duplicate, 100 ml, 200 ml, 500 ml, and 1 liter of HYP culture medium were prepared. The flasks were then inoculated with a suspension of *S.equi* 68222 and incubated-overnight at 37°C while being shaken at 200 rpm. The culture conditions were kept constant for all flasks: 37°C. pH 7, 200 rpm. Once the steady state was reached, optical density at 600 nm and hyaluronic acid production were determined.

As can be seen from the data in Table 3, aeration does not seem to affect bacterial growth very strongly, while good oxygenation is very important for abundant production of hyaluronic acid. It is possible that when aeration is reduced, the available oxygen is used for cell reproduction rather than for biopolymer synthesis.

**TABLE 3**

| Medium vol/flask vol | Final OD 600 | HA g/L |
|---|---|---|
| 1:10 | 4,7 | 1 |
| 1:5 | 4,6 | 0,9 |
| 1:2 | 3,9 | 0,7 |
| 1:1 | 4,5 | 0,6 |

In cultures exposed to strong oxygenation, however, there may be an accumulation of free radicals, i.e., superoxide anions, hydrogen peroxide, hydroxylic radicals, single oxygen, etc. In recent years, it has been suggested that these free radicals may be involved in the degradation of hyaluronic acid (Robert et al. "Treatment of Cartilage Proteoglycan Aggregate With H₂O₂," Biochem. J. 247:349-357 (1987)). It has subsequently been demonstrated that in vivo, the polymer chains of hyaluronic acid are easily degradable both by the effect of physical factors and by chemical factors, such as radiation and oxidizing and enzymatic agents (Merry et al. Annals of the Rheumatic Diseases 48:864-870 (1989); R.A. Greenwald, Seminars of Arthritis and Rheumatism, 20:219-240 (1991)). Other studies have demonstrated that hylauronic acid is depolymerized by the action of free radicals due to an oxidative break-up of the glycoside bond.

The effect of a category of molecules with anti-oxidant characteristics on the action of free radicals and consequently on the depolymerization of hyaluronic acid was therefore assessed.

Of the molecules capable of capturing reactive radical species, there are two types of compounds which have at least one aromatic ring to which are attached one or more hydroxyl groups. These compounds include, for example, 2,3-dihydroxybenzoic acid, salicylic acid, vanillin, tannic acid, etc.

Ten 1 liter flasks were filled with 200 ml of KBS culture medium, and to two of these, 0.11 g/1 of 2,3-hydroxybenzoic acid was added, while salicylic acid, vanillin, and tannic acid were added to other flasks, respectively, at the same concentration. Two flasks were used as controls, and contained only culture medium without any antioxidant molecules.

The flasks were inoculated with a suspension of *S. equi* 68222 so as to obtain an initial optical density of 0.1, and incubated overnight at 37°C while being shaken at 200 rpm. The culture conditions were kept constant in all flasks: 37°C, pH 7, 200 rpm. Once the steady state was reached, fermentation was halted, the hyaluronic acid was purified, and the molecular weight thereof was measured by the GPC-LALLS technique (P. Kratochvil, "Classical Light Scattering From Polymer Solutions, Polymer Science Library, Vol. 5, Elsevier).

The results obtained, shown in Table 4, demonstrate that molecules which act as free radical scavengers can be advantageously used to protect bacterial HA from radical-type degradation, maintaining its molecular weight, thus making such HA useful in a variety of pharmaceutical and other applications.

**TABLE 4**

| Medium Plus Free Radical Scavenger | Final OD 600 | HA g/L | Molecular weight x 10⁶ Da |
|---|---|---|---|
| KBS | 4,8 | 1,2 | 2,56 |
| KBS + tannic acid | 3,75 | 0,9 | 3,1 |
| KBS+vanilin | 5 | 1,2 | 2,65 |
| KBS + gentisic add | 4,25 | 1 | 3,1 |
| KBS + salicylic add | 4,25 | 1 | 3 |

A wide variety of classes of compounds are contemplated for use as free radical scavengers in the medium and methods of the present invention. These include:
Monohydric phenpis:
   Phenol, cresol, xylenol, catechol, 4-methyl-pyrocatechol, urushiol, cresolcinol, and orcinol;
Dihvdric phenols:
Polyhydric phenols:
   Pyrogallol, 1,2,4-benzenetriol, benzenetetraol, and benzenehexaol;
Aromatic hydroxvcarboxvlic acids:
   Gentisinic acid, 2,3-dihydroxybenzoic acid, gallic acid, salicylic acid, protocatechuic acid, pyrogallo-4-carboxylic acid, 3-o-galloylgallic acid, and esters thereof;
Aromatic aldehydes:
   Vanillin, o-vanillin, and protocatechualdehyde;
Heterocyclic compounds:
   Catechin, luteolin, myricetin, and anthocyanidin;
Non-condensed polycyclic compounds having phenolic hydroxyl groups:
   Biphenyldiol and ellagic acid;
Naphthalene compounds having hydroxyl groups:
   Naphthol and naphthalenediol;
Anthracene compounds haying phenolic hydroxyl groups:
   Crysarobin and anthragallol;
Sulfosalicylic acid
Vitamin P
3, 4-dihydroxyphenylalanine
Tannins, Tannic acid

These compounds have been described in EP 0 266 578.

### EXAMPLE 3

*S.equi* 68222 was cultivated in fermenters having a capacity of 3 to 10 liters, fitted with a stirring mechanism, using HYP medium. Since the concentration of oxygen in the culture medium controls hyaluronic acid synthesis as described in Example 2, fermentation was conducted under aerobic conditions so as to keep the concentration of oxygen dissolved in the medium at about 20% saturation. Fermenters were inoculated with a bacterial inoculum equal to 10% (v/v) of the volume of the medium. During fermentation, the temperature was kept at 37°C, and the pH was kept at a value of 7 by adding 0.1N sodium hydroxide. Foaming was controlled by adding a suitable non-toxic anti-frothing agent, such as Silicone 1520 (EU) from Dow Corning. Fermentation lasted about 12 hours. At the end of fermentation, the density of the biomass was equivalent to a turbidity value of 8-12 optical density units measured at 660 nm.

Under these conditions, hyaluronic acid was produced in a yield of about 1.5-2 g/1.

### EXAMPLE 4

### Purification of Hyaluronic Acid From Fermentation Broths

Up to now, various techniques have been employed to purify high molecular weight hyaluronic acid from fermentation broths. Such techniques include treatment with trichloroacetic acid or with a mixture of isoamyl alcohol and chloroform, the use of proteolytic enzymes, precipitation in ethanol or acetone, and selective precipitation of hyaluronic acid with cetylpyridine chloride. These conventional methods for the isolation and purification of hyaluronic acid are unsuitable for industrial purposes because of the many, often complicated, steps required, their high cost, and the toxicity of the reagents involved.

The purification method described below, employing a simple system which does not require the use of toxic reagents and which is particularly cost-effective, facilitates the preparation of hyaluronic acid having a molecular weight of over 2X10⁶ Daltons and a high degree of purity.

At the end of fermentation, the cells are killed by treatment with a bactericide. Alkylating agents such as formaldehyde can be used; which, if used in a 37% aqueous solution, is called formalin. Other useful bactericides include alcohols such as ethyl alcohol and isopropyl alcohol, and organic solvents such as chloroform and acetone. These compounds are normally used at a concentration of 1%, v/v.

To facilitate the release of any hyaluronic acid which may still be attached to the bacterial capsule, a suitable solution of an anionic surfactant, such as sodium dodecyl sulphate, at a concentration of between about 0.02% and about 0.04%, w/v, can be added. Other useful surfactants include, for example, TWEEN 20 (polyoxyethylene sorbitan monolaurate), TWEEN 40 (polyoxyethylene sorbitan monopalmitate), TWEEN 60 (polyoxyethylene sorbitain monostearate), TWEEN 80 (polyoxyethylene sorbitan monooleate), and DIGITONIN.

The bacterial mass is left in contact with these reagents overnight at room temperature, after which the bacterial cells are separated from the culture broth containing the biopolymer. The cells are removed from the culture broth by prefiltration with filters having a porosity of about 0.1 to about 10 microns. Millipore CP15-CP20 filters are particularly appropriate for this purpose. The solution of hyaluronic acid, partially free from bacterial cells, is successfully purified by microfiltration. To eliminate the remaining bacterial cells from the culture broth, tangential flow filtration can be employed. In this method, the fermentation broth is pumped through membranes having a porosity of about 0.45 µm. For example, 0.45 µm Minitan filter plates can be used in conjunction with the Millipore tangential flow filtration system MINITAN-S.

The solution thus obtained is freed from impurities deriving from components of the culture medium, from degradation products of the bacterial cells, from residues of the bactericides used, and from the surfactant agent by means of dialysis against distilled water. For this step, an ultrafiltration membrane with a molecular weight cut-off of 200,000 Daltons can be used. At this point, the filtered solution is dialyzed against between 10 and 50 volumes of distilled water, and the filtrate is discarded. At the end of dialysis, the concentrated solution is freeze-dried. The hyaluronic acid thus obtained has a molecular weight of between about 2 to 3X10⁶ Daltons, measured by the GPC LALLS technique. The purified product is then analysed to determine its protein content and content of nucleic acids and pyrogens. The maximum content of each of these impurities should be: proteins, <1.5%; nucleic acids, <1%; and pyrogens, <10 mg/kg.

### EXAMPLE 5

### Effect of Hyaluronic Acid on Fibroblast Proliferation and Expression of α-Actin

Scar formation is an essential and integral part of tissue repair. Scar tissue confers a certain degree of elasticity to the wound site, allowing the newly-formed tissue to stand up to the stress caused by normal body movements. The deposit of scar tissue is essentially due to the proliferation and production of collagen by some cells (fibroblasts in particular) which migrate to the wound site.

However, as happens in many biological processes, tissue repair can sometimes get out of control and become harmful, leading to serious consequences. In some cases, for example in the case of hypertrophic or keloid wounds, the fibroblasts can be over-stimulated, with the consequent overproduction of granulation tissue. The composition of this granulation tissue may also possess an abnormal ratio between type 1 and type 3 collagen. Moreover, the fibroblasts may produce an excessive quantity of α-actin, thus causing severe contraction of the granulation tissue. This results in the formation of a raised scar in the case of hypertrophic scars, and the formation of a chronic and imperfectly healed scar in the case of keloids.

One approach to the modification of such abnormalities could be the application to the wound site of agents capable of suppressing the proliferation of fibroblasts and the formation of α-actin.

High molecular weight hyaluronic acid, produced according to the method described herein, has been tested to assess its ability to modulate the proliferation of *in vitr*o-cultivated smooth muscle cells of rats, and the expression of α-actin by said cells.

Smooth muscle cells from rat aorta were cultivated in Dulbecco's Modified Eagle's Medium (DMEM). The cells were then placed in 100 mm petri dishes at a concentration of 5X10³ cells/cm². The products to be tested were added to the dishes after 24 hours of culture, and the cells were kept in culture for another seven days. The number of cells was determined using a hemocytometer. The content of smooth α-actin was determined by SDS-PAGE and immunoblotting.

The cells were resuspended in 0.4 M Tris-HC1, pH 6.8, containing 1% SDS, 1% dithiothreitol, 1mM phenylmethylsulfonyl fluoride, 1mM Na-p-tosyl-L-arginine methyl ester, and boiled for three minutes. The protein concentration was determined by Bradford's method (Anal. Biochem. 72:248 (1976)), and between 1 and 5 µg of each sample were loaded onto 5-20% electrophoresis gels. Western blotting was performed as described by Skalli et al. J. Cell Biol. 103:2787 (1991). Modifications in actin expression were assessed by densitometric scanning of the blots.

The results are shown in Tables 5-7.

**TABLE 5**

| Molecular weight (kDa) | Dose (mglmi) | Cellular proliferation* | Expression of α-actin* |
|---|---|---|---|
| <750kd | 0.5 | 118 | 99 |
| -750 kd | 0.5 | 102 | 101 |
| >750kd | 0.5 | 112 | 49 |
| -2x10⁶ kd | 0.5 | 95 | 57 |

| | | | |
|---|---|---|---|
| (^{*}) values are expressed as mean percentages compared to control conditions (10%FCS) | | | |

**TABLE 6**

| Molecular weight (kDa) | Dose (mg/ml) | Cellular proliferation* | Expression of α-actin^{*} |
|---|---|---|---|
| < 750 kd | 2 | 107 | 40 |
| - 750 kd | 2 | 95 | 45 |
| > 750 kd | 2 | 88 | 50 |
| -2x10⁶ kd | 2 | 75 | 20 |

| | | | |
|---|---|---|---|
| (^{*}) values are expressed as mean percentages compared to control conditions (10%FCS) | | | |

**TABLE 7**

| Molecular weight (kDa) | Dose (mglml) | Cellular proliferation* | Expression of a-actin* |
|---|---|---|---|
| <750kd | 5 | 125 | 24 |
| =750 kd | 5 | 82 | 46 |
| >750kd | 5 | 88 | 65 |
| -2X10⁶kd | 5 | 65 | 18 |

| | | | |
|---|---|---|---|
| (^{*}) values are expressed as mean percentages compared to control conditions (10%FCS) | | | |

The cell count and actin levels are expressed as mean percentages compared to the controls. Hyaluronic acid with a molecular weight of 2X10⁶ Daltons inhibits cell proliferation and smooth α-actin expression in a dose-dependent manner.

These results suggest that HA can modulate fibroblast proliferation and contractile activity in hypertrophic and keloid wounds.

### Example 6

### Use of High Molecular Weight Hyaluronic Acid in Wound Healing Modulation and Pharmaceutical Compositions Therefor

Pharmaceutical compositions containing an effective amount of hyaluronic acid having a molecular weight of from about 2X10⁶ Daltons to about 3X10⁶ Daltons, prepared according to the methods of the present invention, alone or in association with one or more active principles, pharmacologically acceptable carriers, diluents, or excipients, can be used to advantage for the reduction of scarring in cases of hypertrophic or keloid scar formation.

Pharmaceutical compositions containing hyaluronic acid adapted for topical, intra-articular, opthalmic, systemic, and peritoneal administration have been described in European Patent 0 138 572 B1. These compositions typically contain hyaluronic acid in an amount of from about 0.1 mg/ml to about 100 mg/ml. For topical and intra-articular administration, they can be in the form of creams, gels, ointments, bandages, and gauzes. For ophthalmic use, they can be in the form of eye drops, lenses, and suture threads. For systemic administration, pharmaceutical preparations for intravenous or intramuscular administration are suitable, such as ampoules or vials.

These pharmaceutical compositions, containing hyaluronic acid having a molecular weight of from about 2X10⁶ Daltons to about 3X10⁶ Daltons, and especially about 2X10⁶ Daltons, can be administered to human or animal subjects to inhibit the formation of hypertrophic scars and keloids arising in at wound sites resulting from injury or surgical intervention. These pharmaceutical compositions can be applied topically to the wound site in the form of a liquid, a cream, a gel, a spray, or a medicated biomaterial, or in the form of an intradermal injection at the time of wound formation or suturing. Doses of high molecular weight hyaluronic acid depend on the individual need of the patient, on the desired effect, and on the route of administration, and can typically be in the range of from about 0.1 mg to about 100 mg per inch or square inch of incision or wound, respectively. The administration of these pharmaceutical compositions to the wound site can be continued on a daily basis as required until such time at the healing process has been completed in order to avoid scar or keloid formation.

The invention being thus described, it will be obvious that the same can be varied in many ways. Such variations are not to be regarded as a departure from the spirit and scope of the invention, and all such modifications as would be obvious to one skilled in the art are intended to be included within the scope of the following claims.

## Claims

1. A culture medium, comprising a soybean meal digest, a peptone mixture and a carbon source,
wherein said soybean meal digest is HY-SOY, said peptone mixture is SPECIAL PEPTONE, and said carbon source is glucose,
said HY-SOY being present at a concentration of between about 10 g/l and about 30 g/l, said SPECIAL PEPTONE being present at a concentration of between about 5 g/l and about 15 g/l, and said glucose being present at a concentration of between about 10 g/l and about 60 g/l.

2. The culture medium of claim 1, further comprising a free radical scavenger.

3. The culture medium of claim 2, wherein said free radical scavenger is at least one member selected from the group consisting of a monohydric phenol, a dihydric phenol, a polyhydric phenol, an aromatic hydroxycarboxylic acid, an aromatic aldehyde, a heterocyclic compound, a non-condensed polycyclic compound having at least one phenolic hydroxyl group, a naphthalene compound having at least one hydroxyl group, an anthracene compound having at least one phenolic hydroxyl group, sulfosalicylic acid, vitamin P, 3,4-dihydroxyphenylalanine, and a tannin.

4. The culture medium of claim 3, wherein said monohydric phenol is selected from the group consisting of phenol, cresol, xylenol, catechol, 4-methyl-pyrocatechol, urushiol, cresolcinol, and orcinol; said polyhyric phenol is a member selected from the group consisting of pyrogallol, 1,2,4-benzenetriol, benzene-tetraol, and benzenehexaol; said aromatic hydroxycarboxylic acid is a member selected from the group consisting of gentisinic acid, 2,3-dihydroxybenzoic acid, gallic acid, salicylic acid, protocatechuic acid, pyrogallo-4-carboxylic acid, and 3-o-galloylgallic acid; said ester of an aromatic hydroxycarboxylic acid is selected from the group consisting of an ester of gentisinic acid, an ester of 2,3-dihydroxybenzoic acid, an ester of gallic acid, an ester of salicylic acid, an ester of protocatechuic acid, an ester of pyrogallo-4-carboxylic acid, and an ester of 3-o-galloylgallic acid; said aromatic aldehyde is selected from the group consisting of vanillin, o-vanillin, and protocatechualdehyde; said heterocyclic compound is selected from the group consisting of catechin, luteolin, myricetin, and anthocyanidin; said non-condensed polycyclic compound having at least one phenolic hydroxyl group is selected from the group consisting of biphenyldiol and ellagic acid; said naphthalene compound having at least one hydroxyl group is selected from the group consisting of naphthol and naphthalenediol; and said anthracene compound having at least one phenolic hydroxyl group is selected from the group consisting of crysarobin and anthragallol.

5. The culture medium of claim 2, wherein said free radical scavenger is present in a concentration of from about 0.01 g/l to about 1 g/l.

6. The culture medium of claim 5, wherein said HY-SOY is present in a concentration of 20 g/l said SPECIAL PEPTONE is present in a concentration of 10 g/l, said glucose is present in concentration of 30 g/l, and said free radical scavenger is present in a concentration of 0.1 g/l, having a pH of between about 6.5 and 7.5.

7. A process for producing hyaluronic acid, comprising:
cultivating a bacterial strain capable of producing hyaluronic acid in a culture medium comprising a soybean meal digest, a peptone mixture, and a carbon source under conditions which promote the formation of hyaluronic acid by said bacterial strain, and recovering said hyaluronic acid,
wherein said soybean meal digest is HY-SOY, said peptone mixture is SPECIAL PEPTONE, and said carbon source is glucose,
said HY-SOY being present at a concentration of between about 10 g/l and about 30 g/l, said SPECIAL PEPTONE being present at a concentration of between about 5 g/l and about 15 g/l, and said glucose being present at a concentration of between about 10 g/l and about 60 g/l.

8. the process of claim 7, wherein said bacterial strain is a strain of Streptococcus.

9. The process of claim 8, wherein said strain of Streptococcus is a group A or group C Streptococcus.

10. The process of claim 8, wherein said strain of Streptococcus is a member selected from the group consisting of S. equi, S, equisimilis, S. dysgalactiae, S. pyogenes, and S. zooepidemicus.

11. The process of claim 10, wherein said S. equi is S. equi 68222.

12. The process of claim 7, wherein said glucose is present at a concentration of 30 g/l.

13. The process of claim 7, wherein said medium further comprises at least one free radical scavenger.

14. The process of claim 13, wherein said free radical scavenger is a member selected from the group consisting of a monohydric phenol, a dihydric phenol, a polyhydric phenol, an aromatic hydroxycarboxylic acid, an aromatic aldehyde, a heterocyclic compound, a non-condensed polycyclic compound having at least one phenolic hydroxyl group, a naphthalene compound having at least one hydroxyl group, an anthracene compound having at least one phenolic hydroxyl group, sulfosalicylic acid, vitamin P, 3,4-dihydroxyphenylalanine, and a tannin.

15. The process of claim 14, wherein said monohydric phenol is selected from the group consisting of phenol, cresol, xylenol, catechol, 4-methyl-pyrocatechol, urushiol, cresolcinol, and orcinol; said polyhyric phenol is a member selected from the group consisting of pyrogallol, 1,2,4-benzenetriol, benzene-tetraol, and benzenehexaol; said aromatic hydroxycarboxylic acid is a member selected from the group consisting of gentisinic acid, 2,3-dihydroxybenzoic acid, gallic acid, salicylic acid, protocatechuic acid, pyrogallo-4-carboxylic acid, and 3-o-galloylgallic acid; said ester of an aromatic hydroxycarboxylic acid is selected from the group consisting of an ester of gentisinic acid, an ester of 2,3-dihydroxybenzoic acid, an ester of gallic acid, an ester of salicylic acid, an ester of protocatechuic acid, an ester of pyrogallo-4-carboxylic acid, and an ester of 3-o-galloylgallic acid; said aromatic aldehyde is selected from the group consisting of vanillin, o-vanillin, and protocatechualdehyde; said heterocyclic compound is selected from the group consisting of catechin, luteolin, myricetin, and anthocyanidin; said non-condensed polycyclic compound having at least one phenolic hydroxyl group is selected from the group consisting of biphenyldiol and ellagic acid; said naphthalene compound having at least one hydroxyl group is selected from the group consisting of naphthol and naphthalenediol; and said anthracene compound having at least one phenolic hydroxyl group is selected from the group consisting of crysarobin and anthragallol.

16. The process of claim 13, wherein said free radical scavenger is present at a concentration of from about 0.01 g/l to about 1 g/l.

17. A process of claims 7 and 16, further comprising:
killing bacterial cells present in said culture medium by treating said cells with a bactericide selected from the group consisting of formaldehye, an alcohol, chloroform, and acetone;
releasing any hyaluronic acid adhering to said bacterial cells by treatment thereof with a surfactant;
removing bacterial cells by prefiltering said culture medium through a filter having a porosity of from about 0.1 µm to about 10 µm;
removing any remaining bacterial cells by microfiltration of said culture medium through a filter having a porosity of about 0.45 µm;
dialyzing said culture medium against distilled water; and
concentrating the dialyzed medium.

## Patentansprüche

1. Kulturmedium, welches einen Sojabohnenmehlverdau, eine Peptongemisch und eine Kohlenstoffquelle umfasst, wobei der Sojabohnemnehlverdau HY-SOY) das Peptongemisch SPECIAL PEPTONE und die Kohlenstoffquelle Glucose ist, wobei HY-SOY in einer Konzentration zwischen etwa 10 g/l und etwa 30 g/l vorhanden ist, SPECIAL PEPTONE in einer Konzentration zwischen etwa 5 g/l und etwa 15 g/l vorhanden ist und die Glucose in einer Konzentration zwischen etwa 10 g/l und etwa 60 g/l vorhanden ist.

2. Kulturmedium nach Anspruch 1, welches weiterhin einen Radikalfänger für freie Radikale umfasst.

3. Kulturmedium nach Anspruch 2, wobei der Radikalfänger für freie Radikale mindestens eine Verbindung ist, welche ausgewählt ist aus einem einwertigen Phenol, einem zweiwertigen Phenol, einem mehrwertigen Phenol, einer aromatischen Hydroxycarbonsäure, einem aromatischen Aldehyd, einer heterocyclischen Verbindung, einer nicht-kondensierten polycyclischen Verbindung mit mindestens einer phenolischen Hydroxylgruppe, einer Naphthalinverbindung mit mindestens einer Hydroxylgruppe, einer Anthracenverbindung mit mindestens einer phenolischen Hydroxylgruppe, Sulfosalicylsäurc, Vitamin P, 3 ,4-Dihydroxyphenylalanin und einem Tannin.

4. Kulturmedium nach Anspruch 3, wobei das einwertige Phenol ausgewählt ist aus Phenol, Cresol, Xylenol, Catechin, 4-Methylpyrocatechin, Urushiol, Cresolcinol und Orcinol, das mehrwertige Phenol eine Verbindung ist, welche ausgewählt ist aus Pyrogallol, 1,2,4-Benzoltriol, Benzoltetraöl und Benzolhexaol, die aromatische Hydroxycarbonsäure eine Verbindung ist, welche ausgewählt ist aus Gentisinsäure, 2,3-Dihydroxybenzoesäüre, Gallussäure, Sallcylsäure, Protocatechusäure, Pyrogallo-4-carbonsäure und 3-o-Galloylgallussäure, der Ester einer aromatischen Hydroxycarbonsäure ausgewählt ist aus einem Gentisitisäurester, einem 2,3-Dihydroxybenzoesäureester, einem Gallusäureester, einem Salicylsäureester, einem Prolocatechusäurester, einem Pyrogallo-4-carbonsäureester und einem 3-o-Galloylgallussäureester, der aromatische Aldehyd ausgewählt ist aus Vanillin, o-Vanillin und Protocatechualdehyd, die heterocyclische Verbindung ausgewählt ist aus Catechin, Luteolin, Myricetin und Anthocyanidin, die nicht-kondensierte polycyclische Verbindung mit mindestens einer phenolischen Hydroxylgruppe ausgewählt ist aus Biphenyldiol und Ellagsäure, die Naphthalinverbindung mit mindestens einer Hydroxylgruppe ausgewählt ist aus Naphthol und Napbthalindiol und die Anthracenverbindung mit nindestens einer phenolischen Hydroxylgruppe ausgewählt ist aus Crysarobin und Anthragallol.

5. Kulturmedium nach Anspruch 2, wobei der Radikalfänger für freie Radikale in einer Konzentralion von etwa 0,01 g/l bis 1 g/l vorhanden ist.

6. Kulturmedium nach Anspruch 5, wobei HY-SOY in einer Konzentration von 20 g/l vorhanden ist, SPECIAL PEPTONE in einer Konzentration von 10 g/l vorhanden ist, die Glucose in einer Konzentration von 30 g/l vorhanden ist und der Radikalfänger für freie Radikale in einer Konzentration von 0,1 g/l vorhanden ist, und welches einen pH-Wert zwischen etwa 6,5 und 7,5 aufweist.

7. Verfahren zur Herstellung von Hyaluronsäure, umfassend:
Kultivieren eines Bakterienstammes, der zur Herstellung von Hyaluronsäure in einem Kulturmedium geeignet ist, welches einen Sojabohnenmeblverdau, ein Peptongemisch und eine Kohlenstoffquelle umfasst, unter Bedingungen, die die Bildung von Hyaluronsäure durch den Bakterienstamm fördern, und Gewinnen der Hyaluronsäure, wobei der Sojabohnenmehlverdau HY-SOY, das Peptongemisch SPECIAL PEPTONE und die Kohlenstoffquelle Glucose ist, wobei HY-SOY in einer Konzentration zwischen etwa 10 g/l und etwa 30 g/l vorhanden ist, SPECIAL PEPTONE in einer Konzentration zwischen etwa 5 g/l und etwa 15 g/l vorhanden ist und die Glucose in einer Konzentration zwischen etwa 10 g/l und etwa 60 g/l vorhanden ist

8. Verfahren nach Anspruch 7, wobei der Bakterienstamm ein Streptokoldenstamm ist.

9. Verfahren nach Anspruch 8, wobei der Streptokokkenstamm ein Streptokokkenstamm der Gruppe A oder Gruppe C ist.

10. Verfahren nach Anspruch 8, wobei der Streptokokkenstamm ausgewählt ist aus S. equi, S. equisimilis, S. dysgalactiae, S. pyogenes und S. zooepidemicus.

11. Verfahren nach Anspruch 10, wobei S. equi die Bedeutung S. equi 68222 hat.

12. Verfahren nach Anspruch 7, wobei die Glucose in einer Konzentration von 30 g/l vorhanden ist.

13. Verfahren nach Anspruch 7, wobei das Medium weiterhin mindestens einen Radikalfänger für freie Radikale umfasst.

14. Verfahren nach Anspruch 13, wobei der Radikalfänger für freie Radikale eine Verbindung ist, welche ausgewählt ist aus einem einwertigen Phenol, einem zweiwertigen Phenol, einem mehrwertigen Phenol, einer aromatischen Hydroxycarbonsäure, einem aromatischen Aldehyd, einer heterocyclischen Verbindung, einer nicht-kondensierten polycyclischen Verbindung mit mindestens einer phenolischen Hydroxylgruppe, einer Naphthalinverbindung mit mindestens einer Hydroxylgruppe, einer Anthracenverbindung mit mindestens einer phenolischen Hydroxylgruppe, Sulfosalicylsäure, Vitamin P, 3,4-Dihydrophenylal anm und einem Tannin.

15. Verfahren nach Anspruch 14, wobei das einwertige Phenol ausgewählt ist aus Phenol, Cresol, Xylenol, Catechin, 4-Methylpyrocatechin, Urushiol, Cresolcinol und Orcinol, das mehrwertige Phenol eine Verbindung ist, welche ausgewählt ist aus Pyrogallol, 1,2,4-Benzoltriol, Benzoltetraol und Benzolhexaol, die aromatische Hydroxycarbonsäure eine Verbindung ist, welche ausgewählt ist aus Gentisinsäure, 2,3-Dihydroxybenzoesäure, Gallussäure, Salicylsäure, Prolocatechusäure, Pyrogallo-4-carbonsäure und 3-o-Galloylgallussäure, der Ester einer aromatischen Hydroxycarbonsäure ausgewählt ist aus einem Gentisinsäurester, einem 2,3-Dihydroxybenzoesäureester, einem Gallussäureester, einem Salicylsäureester, einem Protocatechusäurester, einem Pyrogallo-4-carbonsäureester und einem 3-o-Galloylgallussäureester, der aromatische Aldehyd ausgewählt ist aus Vanillin, o-Vanillin und Protocatechuldehyd, die heterocyclische Verbindung ausgewählt ist aus Catechin, Luteolin, Myricetin und Anthocyanidin, die nicht-kondensierte polycyclische Verbindung mit mindestens einer phenolischen Hydroxylgruppe ausgewählt ist aus Biphenyldiol und Ellagsäure, die Naphthalinverbindung mit mindestens einer Hydroxylgruppe ausgewählt ist aus Naphthol und Naphthalindiol und die Anthracenverbindung mit mindestens einer phenolischen Hydroxylgruppe ausgewählt ist aus Crysarobin und Anthragallol.

16. Verfahren nach Anspruch 13, wobei der Radikalfänger für freie Radikale in einer Konzentration von etwa 0,01 g/l bis 1 g/l vorhanden ist.

17. Verfahren nach Anspruch 7 und 16, welches weiterhin umfasst:
Abtöten der in dem Kulturmedium vorhandenen Bakterienzellen durch Behandlung der Zellen mit einem Bakterizid, welches ausgewählt ist aus Formaldehyd, einem Alkohol, Chloroform, und Aceton,
Freisetzen jeglicher an den Bakterienzellen haftenden Hyaluronsäure durch Behandlung der Zellen mit einem oberflächenaktiven Mittel,
Entfernen der Bakterienzellen durch Vorfilterung des Kulturmediums durch einen Filter mit einer Porösität von etwa 0,1 µm bis etwa 10 µm,
Enfernen jeglicher verbleibender Bakterienzellen durch Mikrofiltration des Kulturmediums durch einen Filter mit einer Porosität yon etwa 0,45 µm,
Dialysieren des Kulturmediums gegen destilliertes Wasser und
Konzentrieren des dialysienen Mediums.

## Revendications

1. Milieu de culture, comprenant un digesté de farine de soja, un mélange de peptones et une source de carbone,
dans lequel ledit digesté de farine de soja est HY-SOY, ledit mélange de peptones est SPECIAL PEPTONE, et ladite source de carbone est le glucose,
ledit HY-SOY étant présent à une concentration comprise entre environ 10 g/l et environ 30 g/l, ledit SPECIAL PEPTONE étant présent à une concentration comprise entre environ 5 g/l et environ 15 g/l, et ledit glucose étant présent à une concentration comprise entre environ 10 g/l et environ 60 g/l.

2. Milieu de culture selon la revendication 1, comprenant de plus un piégeur de radicaux libres.

3. Milieu de culture selon la revendication 2, dans lequel ledit piégeur de radicaux libres est au moins un élément choisi dans le groupe formé par un phénol monohydrique, un phénol dihydrique, un phénol polyhydrique, un acide hydroxycarboxylique aromatique, un aldéhyde aromatique, un composé hétérocyclique, un composé polycyclique non condensé possédant au moins un groupe hydroxyle phénolique, un composé naphtalène possédant au moins un groupe hydroxyle, un composé anthracène possédant au moins un groupe hydroxyle phénolique, de l'acide sulfosalicylique, de la vitamine P, de la 3,4-dihydroxy-phénylalanine, et un tanin.

4. Milieu de culture selon la revendication 3, dans lequel ledit phénol monohydrique est choisi dans le groupe formé par le phénol, le crésol, le xylénol, le catéchol, le 4-méthylpyrocatéchol, l'urushiol, le crésolcinol, et l'orcinol ; ledit phénol polyhydrique est un élément choisi dans le groupe formé par le pyrogallol, le 1,2,4-benzène-triol, le benzènetétraol, et le benzènehexaol ; ledit acide hydroxycarboxylique aromatique est un élément choisi dans le groupe formé par l'acide gentisinique, l'acide 2,3-dihydroxybenzoïque, l'acide gallique, l'acide salicylique, l'acide protocatéchique, l'acide pyrogallo-4-carboxylique, et l'acide 3-o-galloyl-gallique ; ledit ester d'un acide hydroxycarboxylique aromatique est choisi dans le groupe formé par un ester de l'acide gentisinique, un ester de l'acide 2,3-dihydroxybenzoïque, un ester de l'acide gallique, un ester de l'acide salicylique, un ester de l'acide protocatéchique, un ester de l'acide pyrogallo-4-carboxylique, et un ester de l'acide 3-o-galloyl-gallique ; ledit aldéhyde aromatique est choisi dans le groupe formé par la vanilline, l'o-vanilline, et le protocatéchaldéhyde ; ledit composé hétérocyclique est choisi dans le groupe formé par la catéchine, la lutéoline, la myricétine, et l'anthocyanine ; ledit composé polycyclique non condensé possédant au moins un groupe hydroxyle phénolique est choisi dans le groupe formé par le biphényldiol et l'acide ellagique ; ledit composé naphtalène possédant au moins un groupe hydroxyle est choisi dans le groupe formé par le naphtol et le naphtalènediol ; et ledit composé anthracène possédant au moins un groupe hydroxyle phénolique est choisi dans le groupe formé par la crysarobine et l'anthragallol.

5. Milieu de culture selon la revendication 2, dans lequel ledit piégeur de radicaux libres est présent dans une concentration allant d'environ 0,01 g/l à environ 1 g/l.

6. Milieu de culture selon la revendication 5, dans lequel ledit HY-SOY est présent dans une concentration de 20 g/l, ledit SPECIAL PEPTONE est présent dans une concentration de 10 g/l, ledit glucose est présent dans une concentration de 30 g/l, et ledit piégeur de radicaux libres est présent dans une concentration de 0/1 g/l, possédant un pH compris entre environ 6,5 et 7,5.

7. Procédé destiné à produire de l'acide hyaluronique, comprenant:
la culture d'une souche bactérienne capable de produire de l'acide hyaluronique dans un milieu de culture comprenant un digesté de farine de soja, un mélange de peptones, et une source de carbone dans des conditions qui favorisent la formation de l'acide hyaluronique par ladite souche bactérienne, et la récupération dudit acide hyaluronique,
dans lequel ledit digesté de farine de soja est HY-SOY, ledit mélange de peptones est SPÉCIAL PEPTONE, et ladite source de carbone est le glucose,
ledit HY-SOY étant présent à une concentration comprise entre environ 10 g/l et environ 30 g/l, ledit SPECIAL PEPTONE étant présent à une concentration comprise entre environ 5 g/l et environ 15 g/l, et ledit glucose étant présent à une concentration comprise entre environ 10 g/l et environ 60 g/l.

8. Procédé selon la revendication 7, dans lequel ladite souche bactérienne est une souche de *Streptococcus.*

9. Procédé selon la revendication 8, dans lequel ladite souche de *Streptococcus* est un *Streptococcus* du groupe A ou du groupe C.

10. Procédé selon la revendication 8,dans lequel ladite souche de *Streptococcus* est un élément choisi dans le groupe formé par S. *equi, S. equisimilis, S. dysgalactiae, S. pyogenes,* et S. *zooepidemicus.*

11. Procédé selon la revendication 10, dans lequel ledit *S. equi* est *S. equi* 68222.

12. Procédé selon la revendication 7, dans lequel ledit glucose est présent à une concentration de 30 g/l.

13. Procédé selon la revendication 7, dans lequel ledit milieu comprend de plus au moins un piégeur de radicaux libres.

14. Procédé selon la revendication 13, dans lequel ledit piégeur de radicaux libres est un élément choisi dans le groupe formé par un phénol monohydrique, un phénol dihydrique, un phénol polyhydrique, un acide hydroxycarboxylique aromatique, un aldéhyde aromatique, un composé hétérocyclique, un composé polycyclique non condensé possédant au moins un groupe hydroxyle phénolique, un composé naphtalène possédant au moins un groupe hydroxyle, un composé anthracène possédant au moins un groupe hydroxyle phénolique, de l'acide sulfosalicylique, de la vitamine P, de la 3,4-dihydroxyphénylalanine, et un tanin.

15. Procédée selon la revendication 14, dans lequel ledit phénol monohydrique est choisi dans le groupe formé par le phénol, le crésol, le xylénol, le catéchol, le 4-méthylpyrocatéchol, l'urushiol, le crésolcinol, et l'orcinol ; ledit phénol polyhydrique est un élément choisi dans le groupe formé par le pyrogallol, le 1,2,4-benzène-triol, le benzènetétraol, et le benzènehexaol ; ledit acide hydroxycarboxylique aromatique est un élément choisi dans le groupe formé par l'acide gentisinique, l'acide 2,3-dihydroxybenzoique, l'acide gallique, l'acide salicylique, l'acide protocatéchique, l'acide pyrogallo-4-carboxylique, et l'acide 3-o-galloyl-gallique ; ledit ester d'un acide hydroxycarboxylique aromatique est choisi dans le groupe formé par un ester de l'acide gentisinique, un ester de l'acide 2,3-dihydroxybenzoïque, un ester de l'acide gallique, un ester de l'acide salicylique, un ester de l'acide protocatéchique, un ester de l'acide pyrogallo-4-carboxylique, et un ester de l'acide 3-o-galloyl-gallique ; ledit aldéhyde aromatique est choisi dans le groupe formé par la vanilline, l'o-vanilline, et le protocatéchaldéhyde ; ledit composé hétérocyclique est choisi dans le groupe formé par la catéchine, la lutéoline, la myricétine, et l'anthocyanine ; ledit composé polycyclique non condensé possédant au moins un groupe hydroxyle phénolique est choisi dans le groupe formé par le biphényldiol et l'acide ellagique ; ledit composé naphtalène possédant au moins un groupe hydroxyle est choisi dans le groupe formé par le naphtol et le naphtalènediol ; et ledit composé anthracène possédant au moins un groupe hydroxyle phénolique est choisi dans le groupe formé par la crysarobine et l'anthragallol.

16. Procédé selon la revendication 13, dans lequel ledit piégeur de radicaux libres est présent à une concentration allant d'environ 0,01 g/l à environ 1 g/l.

17. Procédé selon les revendications 7 et 16, comprenant de plus:
le fait de tuer des cellules bactériennes présentes dans ledit milieu de culture en traitant lesdites cellules avec un bactéricide choisi dans le groupe formé par du formaldéhyde, un alcool, du chloroforme, et de l'acétone ;
la libération de tout acide hyaluronique collant auxdites cellules bactériennes à l'aide d'un traitement de celles-ci avec un tensioactif ;
l'élimination des cellules bactériennes en préfiltrant ledit milieu de culture à travers un filtre possédant une porosité allant d'environ 0,1 µm à environ 10 µm ;
l'élimination de toutes cellules bactériennes restantes à l'aide d'une microfiltration dudit milieu de culture à travers un filtre possédant une porosité d'environ 0,45 µm ;
la dialyse dudit milieu de culture avec de l'eau distillée ; et
la concentration du milieu dialysé.
